# EUROPEAN PATENT APPLICATION

(11) **EP 4 428 240 A1**
(43) Date of publication of application: **11.09.2024**
(21) Application number: 22887226.3
(22) Date of filing: 31.10.2022
(51) Int. Cl.: C12N 15/80, C12N 1/15, C12P 1/02

(54) **ERYTHRITOL-INDUCIBLE PROMOTER, AND TARGET SUBSTANCE PRODUCTION METHOD USING SAME**

(30) Priority: 01.11.2021 JP 2021178778
(71) Applicant: Kao Corporation, Tokyo 103-8210 (JP)
(72) Inventor: ICHINOSE, Sakurako, Wakayama-shi, Wakayama 640-8580 (JP); SHIBATA, Nozomu, Wakayama-shi, Wakayama 640-8580 (JP); TAKAHASHI, Fumikazu, Wakayama-shi, Wakayama 640-8580 (JP)
(74) Representative: Vossius & Partner Patentanwälte Rechtsanwälte mbB
(86) International application number: PCT/JP2022/040804
(87) International publication number: WO 2023/074901

(57) **Abstract**

An erythritol-inducible promoter consisting of DNA selected from the group consisting of the following (a) to (c): (a) DNA consisting of a nucleotide sequence of any one of SEQ ID Nos: 1 to 4; (b) DNA consisting of a nucleotide sequence having an identity of at least 90% to the nucleotide sequence of any one of SEQ ID Nos: 1 to 4; and (c) DNA consisting of a nucleotide sequence having a deletion, substitution, addition or insertion of one or several nucleotides relative to the nucleotide sequence of any one of SEQ ID Nos: 1 to 4.

## Description

### Field of the Invention

The present invention relates to an erythritol-inducible promoter and a method for producing a target substance using the promoter.

### Background of the Invention

*Trichoderma* fungus can produce a large quantity of an enzyme such as cellulase and xylanase, and has long been drawn attention as a microorganism for producing a cellulolytic enzyme (Non Patent Literature 1). Non Patent Literature 2 discloses that *Trichoderma reesei* is used as a microorganism for producing heterologous proteins derived from a human and other types of microorganisms using its high protein production ability.

Up to the present, as a promoter for inducing gene expression of a target-protein in a *Trichoderma* fungus, a promoter for enolase gene enol and a constitutive expression promoter such as a promoter for translation elongation factor tef1, have been reported (Non Patent Literature 3). However, these promoters are not sufficient for use in the expression of a target substance because their abilities to induce gene expression are not high.

As a *Trichoderma* promoter having a higher ability to induce gene expression, e.g., promoters of cellulase genes cbh1 and cbh2 and a promoter of a xylanase gene xyn3 are used (Non Patent Literature 3). However, these promoters are activated under the conditions of producing cellulase. Because of this, when these promoters are activated, a plurality of cellulase genes present in a *Trichoderma* fungal cell are simultaneously expressed to produce a large amount of cellulase, with the result that a desired protein alone cannot be produced. Production of cellulase can be suppressed by deleting a cellulase gene in a *Trichoderma* fungus, but it is extremely difficult to delete all of the cellulase genes on the genome. In addition, if major cellulase genes are deleted, the growth of a *Trichoderma* fungus under conditions of producing cellulase is significantly decreased.

(Non Patent Literature 1) Kagaku to Seibutsu (Chemistry and Biology), 2012, 50 (8): 592-599, 2012
(Non Patent Literature 2) Appl Biochem Biotechnol, 2011, 165 (5-6): 1169-77
(Non Patent Literature 3) Front Bioeng Biotechnol, 2018, 11 (6): Article 135, doi: 10.3389/fbioe.2018.00135

### Summary of the Invention

In an embodiment, the present invention provides an erythritol-inducible promoter consisting of DNA selected from the group consisting of the following (a) to (c):
(a) DNA consisting of a nucleotide sequence of any one of SEQ ID NOs: 1 to 4;
(b) DNA consisting of a nucleotide sequence having an identity of at least 90% to the nucleotide sequence of any one of SEQ ID NOs: 1 to 4; and
(c) DNA consisting of a nucleotide sequence having a deletion, substitution, addition or insertion of one or several nucleotides relative to the nucleotide sequence of any one of SEQ ID NOs: 1 to 4.

In another embodiment, the present invention provides an expression vector comprising the erythritol-inducible promoter.

In another embodiment, the present invention provides an erythritol-inducible gene expression cassette comprising a gene encoding a target substance or an enzyme involved in synthesis of the target substance and the erythritol-inducible promoter.

In another embodiment, the present invention provides a transformed *Trichoderma* fungal cell comprising the expression vector or the gene expression cassette.

In another embodiment, the present invention provides a method for producing a target substance comprising culturing the transformed *Trichoderma* fungal cell in a culture medium containing erythritol.

In another embodiment, the present invention provides use of DNA selected from the group consisting of (a) to (c) mentioned above as an erythritol-inducible promoter.

### Brief Description of Drawings

FIG. 1 illustrates the relative expression level of 122079 gene after addition of erythritol, glucose or sorbitol. 0h: immediately after addition, 2h: 2 hours after addition.
FIG. 2 illustrates the relative expression level of 68466 gene after addition of erythritol, glucose or sorbitol. 0h: immediately after addition, 2h: 2 hours after addition.
FIG. 3 illustrates the relative expression level of 68606 gene after addition of erythritol, glucose or sorbitol. 0h: immediately after addition, 2h: 2 hours after addition.
FIG. 4 illustrates the relative expression level of 68585 gene after addition of erythritol, glucose or sorbitol. 0h: immediately after addition, 2h: 2 hours after addition.

### Detailed Description of the Invention

All patent literatures, non patent literatures and other publications cited in the specification are incorporated herein in their entirety by reference.

In the specification, the identity of amino acid sequences or nucleotide sequences is calculated in accordance with the Lipman-Pearson method (Science, 1985, 227: 1435-1441); more specifically, calculated using the homology analysis program of genetic information processing software GENETYX Ver. 12 with a unit size to compare (ktup) set to 2.

In the specification, "the identity of at least 90%" used for amino acid sequences or nucleotide sequences refers to an identity of 90% or more, preferably 95% or more, more preferably 96% or more, further preferably 97% or more, further preferably 98% or more, further preferably 99% or more, and further preferably 99.5% or more.

In the specification, "the nucleotide sequence having a deletion, substitution, addition or insertion of one or several nucleotides" refers to a nucleotide sequence having a deletion, substitution, addition or insertion of preferably 1 or more and 10 or less, more preferably 1 or more and 6 or less, further preferably 1 or more and 3 or less, and further preferably 1 or 2 nucleotides. In the specification, the "addition" of a nucleotide includes the addition of a nucleotide to one and both ends of a sequence.

In the specification, the "upstream" and "downstream" regarding a gene refer to the upstream and downstream in the transcription direction, respectively. For example, the "upstream sequence" and "downstream sequence" of a gene refer to, in a DNA sense strand, sequences present on the 5' side and 3' side of the gene, respectively. For example, a "promoter linked upstream of a gene" means that the promoter is present on the 5' side of the gene in a DNA sense strand.

In the specification, the "operable linkage" between a gene and a control region such as a promoter means that the gene and the control region are linked such that the gene can be expressed under the control of the control region. The procedure for creating "operable linkage" between a gene and a control region is commonly known to those skilled in the art.

In the specification, the term "inherent" used for the function, property, and trait of a cell is used to indicate that the function, property, and trait are originally present in the cell. In contrast, the term "foreign" is used to indicate that the function, property, and trait of a cell that are not originally present but introduced from outside. For example, "foreign" nucleotide or DNA refers to a nucleotide or DNA externally introduced into a cell. The foreign nucleotide or DNA may be derived from a homogeneous or heterogenous organism to the cell into which it was introduced (more specifically, a heterogenous nucleotide or heterogenous DNA).

In the specification, the "promoter activity" refers to the activity to promote the transcription of DNA (gene) into mRNA. The promoter activity can be confirmed using an appropriate reporter gene. The promoter activity can be confirmed, for example, by linking DNA encoding a detectable protein, in other words, a reporter gene, downstream of a promoter and measuring the yield of a gene product by the reporter gene. Examples of the reporter gene include genes for enzymes acting on a chromogenic substrate, such as β-galactosidase (LacZ) gene, β-glucuronidase (GUS) gene, luciferase gene, β-lactamase gene and a gene encoding EtbC (2,3-dihydroxy-ethylbenzene 1,2-dioxygenase), and genes for a fluorescent protein, such as a gene encoding GFP (green fluorescent protein). Alternatively, the promoter activity can be confirmed by measuring the expression level of mRNA transcribed from a reporter gene by, e.g., sequencing, quantitative RT-PCR.

In the specification, the "erythritol-inducible promoter" refers to a promoter having promoter activity in the presence of erythritol, preferably, a promoter that can induce expression of mRNA of a target gene (more specifically, a gene to which the promoter is operably linked) in the presence of erythritol at a level 40 times or more, preferably 50 times or more, and more preferably 100 times or more higher than the absence of erythritol or in the presence of cellulose, glucose, or sorbitol; more specifically, refers to a promoter that can induce expression of mRNA of a target gene in the presence of 0.2 w/v% erythritol, 8 hours later, at the level mentioned above.

The present invention provides an erythritol-inducible promoter, an expression vector containing the promoter, a gene expression cassette and a transformed *Trichoderma* fungal cell, and a method for producing a target substance using the transformed *Trichoderma* fungal cell.

The erythritol-inducible promoter provided by the present invention enables induction of the expression of a gene for a target substance in a *Trichoderma* fungus without promoting a cellulase expression induction process inherent to the cell. Accordingly, the present invention makes it possible to produce a target substance with a higher purity in a *Trichoderma* fungus.

It is desired to produce a target substance in a *Trichoderma* fungus without promoting the cellulase production inherent to the cell. The present inventors specified a gene which rarely expresses in the absence of erythritol and whose expression significantly increases in the presence of erythritol in a *Trichoderma* fungus. Erythritol is a kind of sugar alcohol contained in, e.g., fruits and mushrooms. In the culturing of a *Trichoderma* fungus for producing cellulase conventionally performed, mainly cellulose and glucose are used as a carbon source and erythritol is not virtually used. As is shown later in Examples, erythritol does not induce the expression of a cellulolytic enzyme for a biomass inherent to a fungal cell (see, Table 4). Accordingly, when genes for a target substance are expressed by promoters of these genes using erythritol as an induction substance, the target substance can be produced in a *Trichoderma* fungus without promoting the cellulase expression induction process inherent to the cell. The present invention makes it possible to produce a target substance in a *Trichoderma* fungus with a higher purity.

The erythritol-inducible promoter (hereinafter also referred to simply as "the promoter of the present invention") to be provided by the present invention is DNA selected from the group consisting of the following (a) to (c) and having promoter activity in the presence of erythritol.
(a) DNA consisting of a nucleotide sequence of any one of SEQ ID NOs: 1 to 4;
(b) DNA consisting of a nucleotide sequence having an identity of at least 90% to the nucleotide sequence of any one of SEQ ID NOs: 1 to 4; and
(c) DNA consisting of a nucleotide sequence having a deletion, substitution, addition or insertion of one or several nucleotides relative to the nucleotide sequence of any one of SEQ ID NOs: 1 to 4.

The promoters of SEQ ID NOs: 1 to 4 are promoters derived from *Trichoderma reesei.* As mentioned above, since erythritol is not used conventionally for culturing a *Trichoderma* fungus, it has not been known that the promoters of SEQ ID NOs: 1 to 4 are erythritol-inducible promoters. The promoters of SEQ ID NOs: 1 to 4 are erythritol-inducible promoters found for the first time in a *Trichoderma* fungus.

A method for obtaining the promoter of the present invention is not particularly limited. The promoter can be obtained by a chemical synthesis method or a genetic engineering method commonly used. For example, the promoter DNAs of the present invention can be artificially synthesized based on the nucleotide sequences of SEQ ID NOs: 1 to 4. For artificial synthesis of the DNAs, a commercially available DNA synthesis service provided by, for example, GenScript, can be used. Alternatively, the promoter of the present invention can be cloned from a *Trichoderma* fungus such as *Trichoderma reesei.*

The promoter of the present invention can be produced by introducing a mutation into DNA of any one of the nucleotide sequences of SEQ ID NOs: 1 to 4. For introducing a mutation, for example, UV irradiation and site-directed mutagenesis may be used. Examples of the method for site-directed mutagenesis include a method using a splicing overlap extension (SOE) PCR (Gene, 198977: 61-68), an ODA method (Gene, 1995, 152: 271-276) and the Kunkel method (Proc. Natl. Acad. Sci. USA, 1985, 82 (2): 488-492). Alternatively, it is possible to use commercially available kits for site-directed mutagenesis such as Site-Directed Mutagenesis System Mutan-SuperExpress Km kit (Takara Bio Inc.), Transformer^{™} Site-Directed Mutagenesis kit (Clonetech) and KOD-Plus-Mutagenesis Kit (Toyobo Co., Ltd.). The promoter of the present invention can be obtained by selecting DNA having promoter activity in the presence of erythritol from the DNAs into which a mutation has been introduced. For example, the DNA of an erythritol-inducible promoter can be selected by operably linking a reporter gene downstream of the DNAs into which a mutation has been introduced and analyzing the expression level of the reporter gene in the presence of erythritol.

Alternatively, a method for deletion, substitution, addition or insertion of a nucleotide(s) in a nucleotide sequence is, for example, disclosed in Dieffenbach et al., (Cold Spring Harbar Laboratory Press, New York, 581-621, 1995).

Using the aforementioned method, it is possible to obtain erythritol-inducible promoters consisting of the nucleotide sequences of SEQ ID NOs: 1 to 4 or nucleotide sequences having an identity of at least 90% to them, or obtain erythritol-inducible promoters consisting of nucleotide sequences having a deletion, substitution, addition or insertion of one or several nucleotides in the sequences of SEQ ID NOs: 1 to 4.

The promoter of the present invention has the function of controlling the expression of a gene arranged downstream thereof. Using the promoter of the present invention, it is possible to obtain a DNA fragment having an expression control region excellent in transcriptional activity. For example, a DNA fragment containing a target gene and the promoter of the present invention operably linked upstream of the gene can be constructed. The DNA fragment may contain, other than the promoter of the present invention and a target gene, a *cis* element which can increase transcriptional activity of the promoter, or a terminator. The DNA fragment may further contain a selection marker gene such as a drug resistance gene or an auxotrophic marker gene. The DNA fragment containing a target gene and the promoter of the present invention is preferably an erythritol-inducible gene expression cassette for expressing the target gene.

The DNA fragment containing the promoter of the present invention mentioned above can be constructed such that the fragment has a restriction-enzyme recognition sequence at both ends. The promoter of the present invention can be introduced into a vector using the restriction-enzyme recognition sequence. For example, the vector is cleaved with a restriction enzyme and a DNA fragment containing the promoter of the present invention and a restriction-enzyme cleavage sequence at an end is added thereto. In this manner, the promoter of the present invention can be introduced into a vector (restriction enzyme method).

A DNA fragment containing the promoter of the present invention may be introduced directly into the genome of a host cell. A DNA fragment containing the promoter of the present invention may be introduced, for example, upstream of a target gene on the genome of a host cell. Alternatively, a gene expression cassette containing a target gene and the promoter of the present invention as mentioned above may be, for example, introduced into the genome of a host cell.

Alternatively, if the promoter of the present invention is introduced into an expression vector which can express a target gene, it is possible to obtain an expression vector which can increase the expression of the target gene at the transcriptional level. In the expression vector, the promoter of the present invention can be operably linked upstream of DNA encoding a target gene. The expression vector having the promoter of the present invention may be the vector to be introduced into the genome of a host cell or the vector to be retained outside the genome. The vector is preferably replicable in a host cell.

Examples of the vector include pBluescript II SK (-) (Stratagene), pUC vectors such as pUC18/19 and pUC118/119 (Takara Bio Inc.), and pET vector (Takara Bio Inc.), pGEX vector (GE healthcare), pCold vector (Takara Bio Inc.), pHY300PLK (Takara Bio Inc.), pUB110 (Plasmid, 1986, 15 (2): 93-103), pBR322 (Takara Bio Inc.), pRS403 (Stratagene) and pMW218/219 (Nippon Gene Co., Ltd.); pRI vector such as pRI909/910 (Takara Bio Inc.), pBI vector (Clontech), IN3 vector (Inplanta Innovations Inc.), pPTR1/2 (Takara Bio Inc.), pDJB2 (Gene, 1985, 36: 321-331), pAB4-1 (Mol Gen Genet, 1987, 206: 71-75), pLeu4 (Gene, 1989, 84: 335-343), pPyr225 (Mol Genet Genomics, 2002, 268: 397-406), pFG1 (Curr Genet, 1990, 18: 447-451), yeast expression vector pNAN8142 (Biosci Biotechnol Biochem, 1996, 60: 383-389) and pMA91 (Biosci Biotechnol Biochem, 1998, 62: 1615-1618).

In the expression vectors and DNA fragments, the target gene to be arranged downstream of the promoter of the present invention is not particularly limited. The target gene is, for example, a gene encoding a target substance or an enzyme involved in synthesis of the target substance. The target gene may be a heterologous gene encoding a heterologous expression product, a gene derived from the same species and externally introduced, a gene inherently present in a host cell and encoding an expression product, a gene encoding any other protein, peptide or nucleic acid. Examples of the target substance include enzymes, hormones, cytokines, other bioactive peptides, transporters and non-coding RNAs. Examples of the enzymes include oxidoreductases, transferases, hydrolases, lyases, isomerases, and ligases or synthetases. Examples of preferable enzymes include a cellulolytic enzyme for a biomass such as cellulase and hemicellulase, exoglucanase, endoglucanase, β-glucosidase, protease, lipase, mannase, arabinase, galactase and amylase. More preferably, cellulase or hemicellulase is mentioned. Examples of the hemicellulase include xylanase, β-xylosidase and α-arabinofuranosidase. Of them, xylanase is preferable.

The transformant of the present invention can be obtained by introducing an expression vector or DNA fragment containing the promoter of the present invention into a host cell by use of a general transformation method such as an electroporation method, a transformation method, a transfection method, a conjugation method, a protoplast method, a particle gun method or an agrobacterium method.

The host cell into which a vector or a DNA fragment as mentioned above is to be introduced is not particularly limited as long as the promoter of the present invention can function as a promoter in the cell, and is preferably a fungal cell belonging to the genus *Trichoderma.* Examples of the host cell other than this include filamentous fungal cells belonging to the genera *Aspergillus, Penicillium, Neurospora, Fusarium, Chrysosporium, Humicola, Emericella, Hypocrea, Acremonium, Chrysosporium, Myceliophthora, Piromyces, Talaromyces, Thermoascus* and *Thielavia.*

Examples of the fungi belonging to the genus *Trichoderma* include *Trichoderma reesei, Trichoderma longibrachiatum, Trichoderma harzianum, Trichoderma koningii, and Trichoderma viride.* Preferably, *Trichoderma reesei* and a mutant strain thereof are mentioned. For example, *Trichoderma reesei* QM9414 strain and a mutant strain thereof, preferably, *Trichoderma reesei* PC-3-7 strain (ATCC66589), *Trichoderma reesei* PCD-10 strain (FERM P-8172), *Trichoderma reesei* E1AB1 strain (sometimes referred to as JN13 strain) or a mutant strain of any one of them can be used as a host cell. E1AB1 strain is a *Trichoderma reesei* PC-3-7 strain modified such that β-glucosidase (BGL) derived from *Aspergillus aculeatus* is expressed using egl1 promoter (see Enzyme Microb Technol, 2016, 82: 89-95, and WO2013/115305, Examples 1 to 3).

The transformant of the present invention can be used for producing a target substance. For example, a target substance is produced by culturing, in the presence of erythritol, a transformant containing an expression vector or DNA fragment having a gene encoding a target substance or an enzyme involved in synthesis of the target substance and the promoter of the present invention operably linked upstream of the gene to allow the gene to express under the control of the promoter of the present invention. Examples of the target substance are the same as mentioned above.

The culture conditions for a transformant are not particularly limited as long as the transformant cell can grow and a target substance can be produced. As the culture medium for use in culturing, either a synthetic medium or a natural medium may be used as long as it contains components required for ordinary growth of a cell and production of a target substance, such as a carbon source, a nitrogen source, inorganic salts and vitamins. The concentration of erythritol in the culture is preferably from 0.1 to 10% (w/v) as the initial concentration in a culture medium.

As the carbon source to be added to a culture medium, a carbon source not inducing a cellulase is desirably used such that a cellulase expression induction process inherent to a fungal cell is not stimulated. Examples of the carbon source that can be used include carbohydrates not inducing a cellulase such as glucose and fructose, sugar alcohols such as sorbitol, alcohols such as ethanol and glycerol, and organic acids such as acetic acid. Alternatively, erythritol may be used as a carbon source. Meanwhile, preferably, the culture medium does not contain a cellulase-inducible carbon source such as cellulose. To produce a target substance while reducing catabolite repression of a cell as much as possible, the cell may be cultured while continuously feeding a carbon source not inducing a cellulase such as glucose. At this time, it is preferable that the carbon source not inducing a cellulase, e.g., glucose, is dissolved in ammonia water or an aqueous solution containing an ammonium salt serving as a nitrogen source and culturing is carried out by feeding the solution, in consideration of culture efficiency and suppression of bubble formation during the culturing.

Examples of the nitrogen source include ammonia, ammonium salts such as ammonium sulfate, nitrogen compounds such as amine, and natural nitrogen sources such as peptone and a soybean hydrolysate. Examples of the inorganic salts include potassium phosphate, magnesium sulfate, sodium chloride, ferrous sulfate and potassium carbonate. Examples of the vitamins include biotin and thiamine. Furthermore, if necessary, substances required for the growth of the transformant can be added.

Culturing is preferably carried out aerobically by, e.g., shaking culture or an aerated agitation. The culture temperature is preferably 10°C or more, more preferably 20°C or more, and more preferably 25°C or more, and preferably 50°C or less, more preferably 42°C or less and more preferably 35°C or less, in other words, preferably from 10 to 50°C, more preferably from 20 to 42°C, and more preferably from 25 to 35°C. The pH during culturing is from 3 to 9 and preferably from 4 to 5. The culture time is from 10 hours to 10 days and preferably from 2 to 7 days.

After culturing, a target substance is collected from a culture to obtain the target substance. If necessary, the target substance collected can be further purified. A method for collecting or purifying a target substance from a culture is not particularly limited, and the collection and purification may be performed in accordance with a method commonly known collection and purification method. For example, a culture is collected, and, if necessary, subjected to a crushing process for a fungal cell by, e.g., ultrasonic wave or pressurization, and then, subjected to a process such as gradient method, filtration and centrifugation to remove cell components. Thereafter, fractions containing a remaining target substance may be collected. Alternatively, a secretory signal peptide functioning in a transformant is operably linked to a gene encoding the target substance. In this manner, the target substance can be secretory-produced outside the cell.

If necessary, the fractions collected can be subjected to a process such as dialysis, salting out, ion exchange, distillation or solvent extraction or a process employing these in combination to purify a target substance. In the method for producing a target substance according to the present invention, the culturing of a transformant and collection of the target substance can be performed in any one of the batch, semi-batch and continuous systems.

As exemplary embodiments of the present invention, substances, production methods, uses, methods and others will be further disclosed hereinbelow. However, the present invention is not limited to these embodiments.

[1] An erythritol-inducible promoter consisting of DNA selected from the group consisting of the following (a) to (c):
   (a) DNA consisting of a nucleotide sequence of any one of SEQ ID NOs: 1 to 4;
   (b) DNA consisting of a nucleotide sequence having an identity of at least 90% to the nucleotide sequence of any one of SEQ ID NOs: 1 to 4; and
   (c) DNA consisting of a nucleotide sequence having a deletion, substitution, addition or insertion of one or several nucleotides relative to the nucleotide sequence of any one of SEQ ID NOs: 1 to 4.
[2] The erythritol-inducible promoter according to [1], which preferably induces expression of mRNA of a target gene in the presence of erythritol at a level 40 times or more, preferably 50 times or more, and more preferably 100 times or more higher than in the absence of erythritol or in the presence of cellulose, glucose, or sorbitol,
   more preferably induces expression of mRNA of a target gene 8 hours later in the presence of 0.2 w/v% erythritol at a level 40 times or more, preferably 50 times or more, more preferably 100 times or more higher than the absence of erythritol or in the presence of cellulose, glucose, or sorbitol.
[3] An expression vector comprising the erythritol-inducible promoter according to [1] or [2].
[4] The expression vector according to [3], preferably comprising a gene encoding a target substance or an enzyme involved in synthesis of the target substance and the erythritol-inducible promoter linked upstream of the gene.
[5] An erythritol-inducible gene expression cassette comprising a gene encoding a target substance or an enzyme involved in synthesis of the target substance and the erythritol-inducible promoter according to [1] or [2] linked upstream of the gene.
[6] The expression vector according to [3] or [4] or the gene expression cassette according to [5], wherein
   the target substance is preferably an enzyme,
   more preferably, cellulase, hemicellulase, exoglucanase, endoglucanase, β-glucosidase, protease, lipase, mannase, arabinase, galactase, or amylase,
   further preferably, cellulase, xylanase, β-xylosidase, or α-arabinofuranosidase, and
   further preferably cellulase or xylanase.
[7] A transformed *Trichoderma* fungal cell comprising the expression vector according to [3] or [4] or the gene expression cassette according to [5].
[8] The transformed *Trichoderma* fungal cell according to [7], wherein the *Trichoderma* fungus is *Trichoderma reesei* or a mutant strain thereof.
[9] A method for producing a target substance, comprising culturing the transformed *Trichoderma* fungal cell according to [7] or [8] in a culture medium containing erythritol.
[10] The method according to [9], further comprising collecting the target substance from a culture obtained by the culturing.
[11] The method according to [9] or [10], wherein the target substance is preferably an enzyme,
   more preferably, cellulase, hemicellulase, exoglucanase, endoglucanase, β-glucosidase, protease, lipase, mannase, arabinase, galactase or amylase,
   further preferably, cellulase, xylanase, β-xylosidase or α-arabinofuranosidase, and
   further preferably, cellulase or xylanase.
[12] Use of DNA selected from the group consisting of the following (a) to (c) as an erythritol-inducible promoter:
   (a) DNA consisting of a nucleotide sequence of any one of SEQ ID NOs: 1 to 4;
   (b) DNA consisting of a nucleotide sequence having an identity of at least 90% to the nucleotide sequence of any one of SEQ ID NOs: 1 to 4; and
   (c) DNA consisting of a nucleotide sequence having a deletion, substitution, addition or insertion of one or several nucleotides relative to the nucleotide sequence of any one of SEQ ID NOs: 1 to 4.
[13] The use according to [12], wherein
   the erythritol-inducible promoter preferably induces expression of mRNA of a target gene in the presence of erythritol at a level 40 times or more, preferably 50 times or more, and more preferably 100 times or more higher than in the absence of erythritol or in the presence of cellulose, glucose, or sorbitol,
   more preferably induces expression of mRNA of a target gene 8 hours later in the presence of 0.2 w/v% erythritol at a level 40 times or more, preferably 50 times or more, and more preferably 100 times or more higher than in the absence of erythritol or in the presence of cellulose, glucose or sorbitol.
[14] Use of the expression vector according to [4] or the erythritol-inducible gene expression cassette according to [5] for expressing a gene encoding a target substance or an enzyme involved in synthesis of the target substance.
[15] Use of the transformed *Trichoderma* fungal cell according to [7] or [8] for producing a target substance.

### Examples

Now, the present invention will be more specifically described based on Examples but the present invention is not limited to these.

### Example 1 Analysis of expression by RNAseq

*T. reesei* PC-3-7 strain was inoculated in a culture medium such that the number of spores was 1 × 10⁵/mL and subjected to shaking culture at 220 rpm and 28°C (PRXYg-98R manufactured by PRECI Co., Ltd.). The composition of the culture medium was as follows: 3% cellulose, 0.14% (NH₄)₂SO₄, 0.2% KH₂PO₄, 0.03% CaCl₂·2H₂O, 0.03% MgSO₄·7H₂O, 0.1% Bacto Peptone, 0.05% Bacto Yeast extract, 0.1% Tween80, 0.1% Trace element 2, 1.28% diammonium hydrogen citrate, a 50 mM tartrate buffer (pH4.0) (all % indicate w/v%) . The composition of Trace element 2 was as follows: 6 mg H₃BO₃, 26 mg (NH₄)₆Mo₇O₂₄·4H₂O, 100 mg FeCl₃·6H₂O, 40 mg CuSO₄·5H₂O, 8 mg MnCl₂·4H₂O and 200 mg ZnCl₂, which were diluted with distilled water up to 100 mL. After culturing was carried out for 72 hours in the above conditions, erythritol was added to the culture medium so as to obtain a final concentration of 0.2 w/v%. After culturing was carried out for further 8 hours, fungal cells were collected. Fungal cells cultured in the absence of erythritol for 80 hours in the above conditions were used as a control.

Total RNA was extracted from the fungal cells collected (using RNeasy Plant Mini Kit manufactured by QIAGEN). The total RNA extracted was subjected to a treatment to be performed by TruSeq RNA sample Prep v3 kit (Illumina) to construct a next-generation sequencer library (cDNA library). The cDNA library obtained was subjected to sequencing to be performed by the Miseq system using Miseq Reagent Kit (300 cycles) (Illumina). The sequence information obtained was mapped by use of CLC Genomics Workbench to the CDS sequence of *T. reesei* QM6a obtained from the Ensembl Fungi database (fungi.ensembl.org/Trichoderma_reesei/Info/Index/). RPKM (Reads Per Kilobase of exon per Million mapped reads), which is a value obtained by normalizing the number of mapped reads by the length of the gene and total number of reads, was obtained for individual genes. Based on the RPKM obtained, the expression levels of individual genes were analyzed. As a result, the genes, which were found, based on the RPKM value, to exhibit 50 times or more higher expression in the erythritol-adding condition than the control but exhibit virtually no expression in the control condition, were the following 4 genes: 122079 (TRIREDRAFT_122079), 68466 (TRIREDRAFT_68466), 68606 (TRIREDRAFT_68606) and 68585 (TRIREDRAFT_68585) (Table 1).

**[Table 1]**

| Gene ID | Putative function | Control (Cont) | Presence of erythritol (Ery) | Rate (Ery/Cont) |
|---|---|---|---|---|
| TRIREDRAFT_123989 | glycoside_hydrolase_family_7 (CBH1) | 84315 | 57860 | 0.69 |
| TRIREDRAFT_72567 | glycoside_hydrolase_family_6 (CBH2) | 81284 | 47275 | 0.58 |
| TRIREDRAFT_120961 | glycoside_hydrolase_family_61 (EG7) | 55928 | 24059 | 0.43 |
| TRIREDRAFT_122081 | glycoside_hydrolase_family_7 (EG1) | 23758 | 16909 | 0.71 |
| TRIREDRAFT_120312 | glycoside_hydrolase_family_5 (EG2) | 22140 | 15903 | 0.72 |
| TRIREDRAFT_73638 | carbohydrate-binding_module_family_1 (CIP1) | 19181 | 12067 | 0.63 |
| TRIREDRAFT_68585 | galactose-6-phosphate_isomerase | 0 | 9417 | >100 |
| TRIREDRAFT_49976 | glycoside_hydrolase_family_45 (EG5) | 16317 | 7736 | 0.47 |
| TRIREDRAFT_23232 | glycoside_hydrolase_family_12 (EG3) | 13023 | 6029 | 0.46 |
| TRIREDRAFT_20749 | glycoside_hydrolase_family_1 (BGL2) | 6033 | 6007 | 1.0 |
| TRIREDRAFT_73632 | carbohydrate_esterase_family_5 (AXE1) | 7314 | 5773 | 0.79 |
| TRIREDRAFT_68466 | glycerone_kinase | 0 | 4336 | >100 |
| TRIREDRAFT_20229 | glycoside_hydrolase_family_10 (XYN3) | 10083 | 3760 | 0.37 |
| TRIREDRAFT_22079 | predicted_protein | 54 | 3543 | 66 |
| TRIREDRAFT_68606 | triose-phosphate_isomerase | 14 | 894 | 66 |

### Example 2 Analysis for expression of erythritol-inducible expression gene

The erythritol specificity of the 4 genes found in Example 1 in expression induction was examined by a real-time PCR. In the examination, *T. reesei* E1AB1 strain (Enzyme Microb Technol, 2016, 82: 89-95) was used, which was obtained by modifying *T. reesei* PC-3-7 strain such that AaBGL1 was highly expressed by egl1 promoter. E1AB1 strain was inoculated such that the number of spores was 1 × 10⁵/mL and subjected to shaking culture at 220 rpm and 28°C (PRXYg-98R manufactured by PRECI Co., Ltd.). The composition of the culture medium was as follows: 1% glucose, 0.14% (NH₄)₂SO₄, 0.2% KH₂PO₄, 0.03% CaCl₂·2H₂O, 0.03% MgSO₄·7H₂O, 0.1% Bacto Peptone, 0.05% Bacto Yeast extract, 0.1% Tween80, 0.1% Trace element 2, 1.28% diammonium hydrogen citrate and a 50 mM tartrate buffer (pH4.0) (all % indicate w/v%). The composition of Trace element 2 was the same as employed in Example 1. After culturing was carried out for 48 hours in the above conditions, erythritol, glucose or sorbitol was added to the culture medium so as to obtain a final concentration of 0.2 w/v%. Immediately after (0h) and 2 hours after (2h) the addition of erythritol, fungal cells were collected. Fungal cells cultured for 50 hours in total without adding any carbon source after the 48-hour culture, were used as a control.

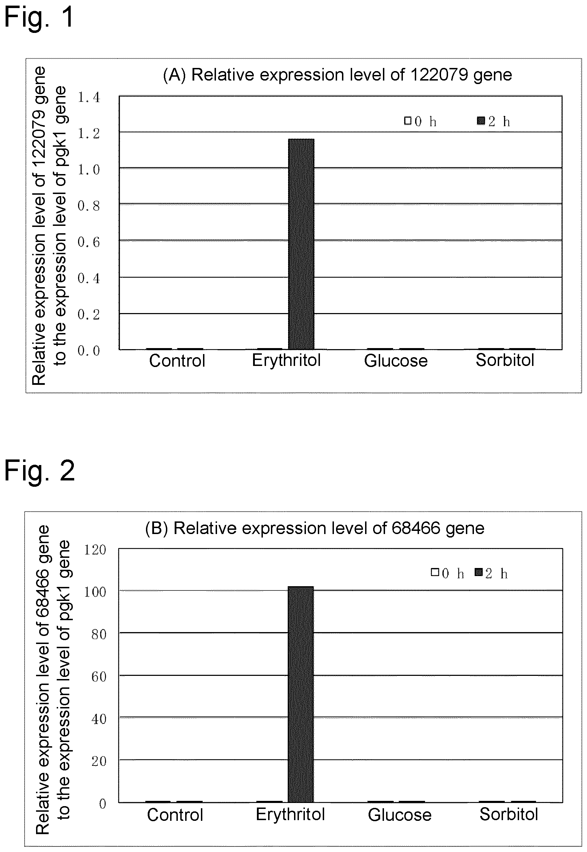

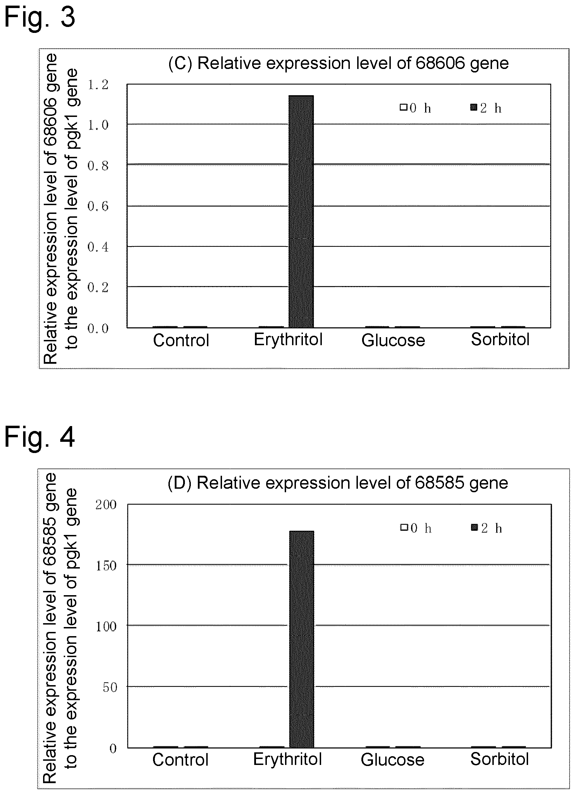

Using RNA extracted from the fungal cells collected, cDNA was synthesized (using PrimeScript^{™} II 1st strand cDNA Synthesis Kit manufactured by TaKaRa Bio Inc.). The cDNA synthesized was subjected to real-time PCR analysis (using Brilliant III Ultra-Fast SYBR Green QPCR Master Mixes of Agilent Technologies). The primers used in the real-time PCR are shown in Table 2. The expression levels of individual genes were obtained in accordance with a relative quantification procedure (ΔΔ Ct method). A phosphoglycerate kinase (a kind of glycolytic enzyme) gene, pgk1 (TRIREDRAFT_21406) was used as normalization gene. Relative expression levels of individual genes to the expression level of pgk1 gene are shown in Figures 1 to 4. The expression levels of 4 genes significantly increased 2 hours (2h) after addition of erythritol compared to immediately (0h) after addition. In contrast, the expression levels of the 4 genes did not increase in the control and in glucose or sorbitol-adding condition. From this, it was demonstrated that the expression of 4 genes: 122079, 68466, 68606 and 68585 is induced in an erythritol-specific manner.

**[Table 2]**

| Target gene | Primer (5'→3' sequence) | | SEQ ID NO. |
|---|---|---|---|
| 122079 | Fw | CGGTGGTGGAAGAGGATTG | 5 |
| | Rv | AGACGGATCAGGCAGGATGT | 6 |
| 68466 | Fw | TGGACACACTCATCCCGTTT | 7 |
| | Rv | CTCTTCCCAGTCTCGGCTTC | 8 |
| 68606 | Fw | ACCTGCTGGAGAGAAGCACA | 9 |
| | Rv | CCCCATACAAAACCCTCGTC | 10 |
| 68585 | Fw | GTCTAGGCGTCGCCATTTCT | 11 |
| | Rv | CAACTTCCAGCCCAACAACC | 12 |
| pgk1 | Fw | TCTGGGCGACATCTACAT | 13 |
| | Rv | GTAGTCGAGCTCCTTCTTCA | 14 |

### Example 3 Construction of plasmid DNA to be introduced into gene

Whether the promoter regions of the 4 genes, which were confirmed to induce expression in an erythritol-specific manner in Example 2, function as the erythritol-inducible promoter was examined. The following 6 DNA fragments were prepared by a PCR using genomic DNA of *T. reesei* as a template.
Fragment 1: promoter region at about 1.0 kbp upstream of 122079 gene (SEQ ID NO: 1)
Fragment 2: promoter region at about 0.8 kbp upstream of 68466 gene (SEQ ID NO: 2)
Fragment 3: promoter region at about 0.8 kbp upstream of 68606 gene (SEQ ID NO: 3)
Fragment 4: promoter region at about 0.8 kbp upstream of 68585 gene (SEQ ID NO: 4)
Fragment 5: region ranging from a coding region of xylanase gene xyn 3 (TRIREDRAFT_120229) to 0.5 kbp downstream of the gene
Fragment 6: region (about 2.4 kbp) from about 1.0 kbp upstream of pyr4 gene (TRIREDRAFT_74020) to ORF and about 0.3 kbp downstream of the gene

The primers used for amplification of the fragments are shown in Table 3.

**[Table 3]**

| | Name of fragment | Length of fragment | Primer (5'→3' sequence) | | SEQ ID NO. |
|---|---|---|---|---|---|
| Fragment 1 | P122079 | about 1.0 kb | Fw | ATTATACGAACGGTACTTCGACATCAAAATCACCT | 15 |
| | | | Rv | GACGTTTGCTTTCATGTTTTGATAAGGAGCGGTAG | 16 |
| Fragment 2 | P68646 | about 0.8 kb | Fw | ATTATACGAACGGTACGTATATGTCTATCAGTGAT | 17 |
| | | | Rv | GACGTTTGCTTTCATTGTTCTATTCTGTAGAATGT | 18 |
| Fragment 3 | P68606 | about 0.8 kb | Fw | ATTATACGAACGGTATGGATGACACGGTAGACAAG | 19 |
| | | | RV | GACGTTTGCTTTCATTTTGGTCGAGAGGCCGATGA | 20 |
| Fragment 4 | P68585 | about 0.8 kb | Fw | ATTATACGAACGGTATGTTCTATTCTGTAGAATGT | 21 |
| | | | Rv | GACGTTTGCTTTCATCGTATATGTCTATCAGTGAT | 22 |
| Fragment 5 | xyn3-Txyn3 | about 1.9 kb | Fw | ATGAAAGCAAACGTCATCTT | 23 |
| | | | RV | ACAACGAGAAAAGAAGTCTCTGGCTGAGTGCTCGT | 24 |
| Fragment 6 | pyr4 | about 2.4 kb | Fw | TTCTTTTCTCGTTGTCTCACTCT | 25 |
| | | | RV | AGATCCACCAGATATGTCAGGA | 26 |

Fragments 5 and 6 were ligated to prepare XYN3-pyr4 and then XYN3-pyr4 was ligated to each of Fragments 1 to 4. The obtained fragments were each inserted into pUC118 (Takara Bio Inc.) at a cleavage site with HincII restriction enzyme to construct 4 vectors: pUC-P122079-XYN3-pyr4, pUC-P68466-XYN3-pyr4, pUC-P68606-XYN3-pyr4 and pUC-P68585-XYN3-pyr4. DNA fragments were ligated in accordance with the protocol of In-Fusion HD Cloning kit (Takara Bio Inc.).

*E. coli* DH5α Competent Cells (Takara Bio Inc.) were transformed with the plasmids constructed. Among the transformants obtained as ampicillin-resistant strains, strains containing a plasmid into which a target gene had been inserted were selected by a colony PCR. The selected transformants were cultured in LB medium containing ampicillin (37°C, one day). From the fungal cells obtained, plasmids were collected and purified by NucleoSpin Plasmid EasyPure (Macherey-Nagel).

### Example 4 Preparation of transformed strain

*T. reesei* E1AB1Δpyr4 strain was transformed with each of the plasmids constructed in Example 3. Introduction was carried out in accordance with the protoplast PEG method (Biotechnol Bioeng, 2012, 109 (1): 92-99). Transformants were screened using pyr4 gene as a selection marker in a selective medium (2% glucose, 1.1 M sorbitol, 2% agar, 0.5% (NH₄)₂SO₄, 0.2% KH₂PO₄ (pH5.5), 0.06% CaCl₂·2H₂O, 0.06% CsCl₂, 0.06% MgSO₄·7H₂O, 0.1% Trace element 1; all % indicate w/v%). The composition of Trace element 1 was as follows: 0.5 g FeSO₄·7H₂O, 0.2 g CoCl₂, 0.16 g MnSO₄·H₂O, 0.14 g ZnSO₄·7H₂O, which were diluted with distilled water up to 100 mL. The transformants screened were stabilized by subculturing and thereafter strains stably containing a target gene were further selected by a colony PCR.

### Example 5 Culturing of transformed strain

The transformed strains prepared in Example 4 were cultured to produce proteins by erythritol induction. In the culturing, 50 mL of culture medium was placed in a 500 mL-flask. Spores of the strains prepared in Example 4 were inoculated in the culture medium so as to obtain a density of 1 × 10⁵/mL and subjected to shaking culture at 220 rpm and 28°C (PRXYg-98R manufactured by PRECI Co., Ltd.). The composition of the culture medium was as follows: 1% glucose, 0.14% (NH₄)₂SO₄, 0.2% KH₂PO₄, 0.03% CaCl₂·2H₂O, 0.03% MgSO₄·7H₂O, 0.1% Bacto Peptone, 0.05% Bacto Yeast extract, 0.1% Tween80, 0.1% Trace element 2, 50 mM tartrate buffer (pH4.0) (all % indicate w/v%). The composition of Trace element 2 was the same as that employed in Example 1. Forty-eight hours after culturing, erythritol was added so as to obtain a final concentration of 0.2 w/v% and culture solutions were collected. Culturing was carried out for further 4 hours, and thereafter, the culture solutions were collected again.

### Example 6 Measurement of XYN3 activity

The activity of xylanase (XYN3) in the culture solutions collected in Example 5 was measured in accordance with the pNP (p-nitrophenol) method. The culture supernatants were diluted to prepare enzyme solutions. A 1 mM pNP-β-xylobioside solution (50 mM Na-acetate buffer, pH5.0) was used as a substrate solution. To each of the enzyme solutions (20 µL), the substrate solution (80 µL) was added. After a reaction was carried out at 50°C for 10 minutes, 100 µL of a 1 M Na₂Co₃ solution was added to terminate the reaction. Thereafter, the absorbance at 420 nm was measured. The same operation was carried out using p-nitrophenol to prepare a calibration curve. The amount of an enzyme to separate 1 µmol of pNP in a minute was defined as 1 U and the activity of XYN3 in each of the culture solutions was obtained.

As shown in Table 4, in the parent E1AB1 strain, XYN3 activity was not virtually found 4 hours after addition of erythritol, whereas, in 122079 promoter expression strain (P122079), 68466 promoter expression strain (P68466) and 68585 promoter expression strain (P68585), it was found that XYN3 activity significantly increased 4 hours after addition of erythritol compared to immediately after (0h) addition. From these results, it was demonstrated that these promoters function as an erythritol-inducible promoter and enable induction-production of a target substance in the presence of erythritol. Since parent E1AB1 strain exhibited no XYN3 activity in erythritol-adding condition, it was demonstrated that erythritol does not induce the expression of cellulolytic enzyme for a biomass inherent to the fungal cell. Since it was confirmed that expression of 68606 gene is induced by erythritol in Examples 1 and 2, it is clear that 68606 promoter functions as an erythritol-inducible promoter. In contrast, the 68606 promoter expressing strain (P68606) did not show XYN3 activity in the presence of erythritol. This was considered that the experimental system was not suitable for P68606 strain.

**[Table 4]**

| Fungus strain | Erythritol | 0 h (U/ml) | 4 h (U/ml) |
|---|---|---|---|
| Parent strain | - | 0 | 0 |
| | + | 0 | 0 |
| P122079 | - | 0.300 | 0.335 |
| | + | 0.294 | 1.24 |
| P68466 | - | 0 | 0 |
| | + | 0 | 1.55 |
| P68606 | - | 0 | 0 |
| | + | 0 | 0 |
| P68585 | - | 0 | 0 |
| | + | 0 | 1.23 |

## Claims

1. An erythritol-inducible promoter consisting of DNA selected from the group consisting of the following (a) to (c):
(a) DNA consisting of a nucleotide sequence of any one of SEQ ID NOs: 1 to 4;
(b) DNA consisting of a nucleotide sequence having an identity of at least 90% to the nucleotide sequence of any one of SEQ ID NOs: 1 to 4; and
(c) DNA consisting of a nucleotide sequence having a deletion, substitution, addition or insertion of one or several nucleotides relative to the nucleotide sequence of any one of SEQ ID NOs: 1 to 4.

2. An expression vector comprising the erythritol-inducible promoter according to claim 1.

3. The expression vector according to claim 2, comprising a gene encoding a target substance or an enzyme involved in synthesis of the target substance and the erythritol-inducible promoter linked upstream of the gene.

4. An erythritol-inducible gene expression cassette comprising a gene encoding a target substance or an enzyme involved in synthesis of the target substance and the erythritol-inducible promoter according to claim 1 linked upstream of the gene.

5. A transformed *Trichoderma* fungal cell comprising the expression vector according to claim 2 or 3 or the gene expression cassette according to claim 4.

6. The transformed *Trichoderma* fungal cell according to claim 5, wherein the *Trichoderma* fungus is *Trichoderma reesei* or a mutant strain thereof.

7. A method for producing a target substance, comprising culturing the transformed *Trichoderma* fungal cell according to claim 5 or 6 in a culture medium containing erythritol.

8. The method according to claim 7, further comprising collecting the target substance from a culture obtained by the culturing.

9. Use of DNA selected from the group consisting of the following (a) to (c) as an erythritol-inducible promoter:
(a) DNA consisting of a nucleotide sequence of any one of SEQ ID NOs: 1 to 4;
(b) DNA consisting of a nucleotide sequence having an identity of at least 90% to the nucleotide sequence of any one of SEQ ID NOs: 1 to 4; and
(c) DNA consisting of a nucleotide sequence having a deletion, substitution, addition or insertion of one or several nucleotides relative to the nucleotide sequence of any one of SEQ ID NOs: 1 to 4.

10. The use according to claim 9, wherein the erythritol-inducible promoter induces expression of mRNA of a target gene in the presence of erythritol at a level 40 times or more, preferably 50 times or more, and more preferably 100 times or more higher than in the absence of erythritol or in the presence of cellulose, glucose, or sorbitol.

11. Use of the expression vector according to claim 3 or the erythritol-inducible gene expression cassette according to claim 4 for expressing a gene encoding a target substance or an enzyme involved in synthesis of the target substance.

12. Use of the transformed *Trichoderma* fungal cell according to claim 5 or 6 for producing a target substance.
